# EUROPEAN PATENT APPLICATION

(11) **EP 4 527 494 A1**
(43) Date of publication of application: **26.03.2025**
(21) Application number: 24190066.1
(22) Date of filing: 22.07.2024
(51) Int. Cl.: B01F 33/501, A61J 1/20, A61M 5/315, A61M 5/34

(54) **FREEZE-DRY POWDER INJECTOR RESTORING DEVICE**

(30) Priority: 16.01.2024 TW 113101681
(71) Applicant: Chen, Chang-Tzu, Taipei (TW)
(72) Inventor: CHEN, CHANG-TZU, Taipei City (TW); WANG, MING-SHEN, New Taipei City (TW); CHANG, KIN-SEN, New Taipei City (TW)
(74) Representative: Casalonga

(57) **Abstract**

A freeze-dry powder injector restoring device comprises a container (10, 10D), a piston (20, 20C, 20D), and a top cover (30). The container (10, 10D) has a lower connecting portion (11, 11D), a cup body (12, 12D), and a tube portion (13, 13D). The tube portion (13, 13D) is in the cup body (12, 12D). The tube portion (13, 13D) has a tube opening (131). The piston (20, 20C, 20D) divides the inner space into a first chamber (14) and a second chamber (15). Multiple communicating channels (17) are formed between the first chamber (14) and the second chamber (15). When the tube opening (131) is open, the second chamber (15) fluidly communicates with the first chamber (14). The freeze-dry powder injector restoring device meets a requirement of freeze-drying locally in the original container (10, 10D) and assembling on the production line.

## Description

### 1. Field of the Invention

The present invention relates to a medical device, especially to a freeze-dry powder injector restoring device that is configured to accommodate and restore freeze-dried medicinal powder.

### 2. Description of the Prior Arts

Freeze-drying is a common method to preserve medicine that is capable of maintaining stability of the active ingredient such as chemical stability, physical stability, and biological stability, and resolving issues of instability of pharmaceutical aqueous solution and structural destruction of the medicine by heat, thereby stabilizing active ingredient in the medicine and thus a period before expiration may be extended. Some injections may be produced as freeze-dried medicinal powder, and those injections need to be dissolved before giving injection.

However, the process of dissolving the freeze-dried medicinal powder injection is complicated which includes more than ten steps and involves with several relative tools. For those medical personnel who might conduct hundreds of injections, the process of dissolving the freeze-dried medicinal powder injection would take too much time and effort. In addition, operation errors and dispensing oversights may occur during dissolving the freeze-dried medicinal powder injection since the process is complicated.

A dual chamber mixing syringe has been created for mixing different kinds of medicines, but it is difficult to fill the freeze-dried medicinal powder injection and the solvent simultaneously in the dual chamber mixing syringe, and the filling process would be complicated. Besides, the preservation period of the freeze-dried medicinal powder injection will be shortened and an overall weight is gained due to the solvent, and hence the advantages of weighing less and long preservation period from freeze-dried medicinal powder are lost.

In addition, some of the dual chamber mixing syringes have the chamber containing the freeze-dried medicinal powder to be arranged at a liquid outlet of the needle, and thus once the freeze-dried medicinal powder is not completely dissolved, the undissolved freeze-dried medicinal powder may be stuck in the needle such that the whole syringe is out of use. Furthermore, some of the dual chamber mixing syringes lack mechanisms helping dissolve the freeze-dried medicinal powder when a dissolving speed is slow during mixing the freeze-dried medicinal powder and the solvent.

To overcome the shortcomings, the present invention provides a freeze-dry powder injector restoring device to mitigate or obviate the aforementioned problems.

The main objective of the present invention is to provide a freeze-dry powder injector restoring device that has a brand-new design to have every component simplified and can be manufactured easily, and thus has a better manufacturing yield. The freeze-dry powder injector restoring device would be easy to sterilize to achieve a requirement of cleanness and sterility, and matches freeze-dried medicine producing procedures to meet a requirement of freeze-dried locally in the original container and assembling on the production line. Besides, the freeze-dry powder injector restoring device has a mechanism which leads and changes a direction of a solvent entering a chamber, to prevent the freeze-dried medicinal powder stuck in the needle during dissolving.

The freeze-dry powder injector restoring device includes a container, a piston, and a top cover. The container has a lower connecting portion, a cup body, and a tube portion. The lower connecting portion is located at an end of the container, and the lower connecting portion forms a lower opening. The cup body is located at another end of the container, and the cup body forms an inner space and an upper opening which fluidly communicate with each other. The tube portion is mounted in the cup body, an end of the tube portion fluidly communicates with the lower opening, and another end of the tube portion forms a tube opening. The piston is mounted in the container and divides the inner space into a first chamber and a second chamber, and the piston has a separating board and a connecting sleeve. The connecting sleeve protrudes from a side of the separating board, the connecting sleeve is mounted on the tube portion and adjacent to the tube opening, and the connecting sleeve is capable of sliding along the tube portion. The connecting sleeve has a communicating opening and multiple communicating channels which are formed on the piston and disposed between the first chamber and the second chamber. The top cover has an upper connecting portion and a covering portion. The upper connecting portion is located at a side of the top cover. The upper connecting portion forms a connecting opening which fluidly communicates with the first chamber. The covering portion is located at another side of the top cover. The covering portion surrounds and protrudes from an outer wall surface of the upper connecting portion. The top cover is fixed at the upper opening of the cup body via the covering portion. When the tube opening is closed by the piston, the communicating opening and each one of the communicating channels is closed, and when the tube opening is open, the tube opening, the communicating opening, the second chamber, each one of the communicating channels, and the first chamber fluidly communicate with each other.

Since the freeze-dried medicinal powder has been accommodated in the container, the freeze-dry powder injector restoring device is capable of directly combining with the needle and the syringe, such that procedures of dissolving the freeze-dried medicinal powder can be simplified to increase convenience and efficiency of injection for patients or medical personnel, and it is convenient for the patient or medical personnel to carry the freeze-dried medicinal powder in the freeze-dry powder injector restoring device. The process, steps, time, and manpower needed for dissolving the freeze-dried medicinal powder may be simplified or reduced thanks to the freeze-dry powder injector restoring device. In addition, because a quantity of the freeze-dried medicinal powder is fixed, a risk of dosage misjudgment would be reduced. Furthermore, the freeze-dry powder injector restoring device may further have a mixer helping dissolve the freeze-dried medicinal powder.

As to the medicine maker, the freeze-dry powder injector restoring device is easy to manufacture since every component of the freeze-dry powder injector restoring device is easy to make, and thus the freeze-dry powder injector restoring device has a better manufacturing yield. The freeze-dry powder injector restoring device is easy to sterilize to achieve a requirement of cleanness and sterility. In addition, the freeze-dry powder injector restoring device matches freeze-dried medicine producing procedures to meet a requirement of freeze-drying locally in the original container and assembling on the production line, and thereby a cost of medicine manufacturing can be reduced.

Other objectives, advantages and novel features of the invention will become more apparent from the following detailed description when taken in conjunction with the accompanying drawings.

### In the drawings:

Fig. 1 is a perspective view of a first embodiment of a freeze-dry powder injector restoring device in accordance with the present invention;
Fig. 2 is an exploded perspective view in partial section of the freeze-dry powder injector restoring device in Fig. 1;
Fig. 3 is another exploded perspective view in partial section of the freeze-dry powder injector restoring device in Fig. 1, shown in another view angle;
Fig. 4 is a cross-sectional side view in partial section of the freeze-dry powder injector restoring device in Fig. 1, showing a tube opening closed;
Fig. 5 is another cross-sectional side view in partial section of the freeze-dry powder injector restoring device in Fig. 1, showing the tube opening open;
Fig. 6 is a cross-sectional top side view of the freeze-dry powder injector restoring device in Fig. 1;
Figs. 7 to 9 are serial figures showing filling a liquid state injection and assembling of the freeze-dry powder injector restoring device in Fig. 1;
Fig. 10 is a cross-sectional side view in partial section of the freeze-dry powder injector restoring device in Fig. 1, showing assembling with a needle and a syringe;
Fig. 11 is an operational side view in partial section of the freeze-dry powder injector restoring device in Fig. 1, shown in use;
Fig. 12 is an exploded view in partial section of a second embodiment of a freeze-dry powder injector restoring device in accordance with the present invention;
Fig. 13 is another exploded view in partial section of the freeze-dry powder injector restoring device in Fig. 12, shown in another view angle;
Fig. 14 is a cross-sectional side view in partial section of the freeze-dry powder injector restoring device in Fig. 12, showing the tube opening closed;
Fig. 15 is a cross-sectional side view in partial section of the freeze-dry powder injector restoring device in Fig. 12, showing the tube opening open;
Fig. 16 is a perspective view of a third embodiment of a freeze-dry powder injector restoring device in accordance with the present invention;
Fig. 17 is an exploded perspective view in partial section of the freeze-dry powder injector restoring device in Fig. 16;
Fig. 18 is another exploded perspective view of the freeze-dry powder injector restoring device in Fig. 16, shown in another view angle;
Fig. 19 is a cross-sectional side view in partial section of the freeze-dry powder injector restoring device in Fig. 16;
Fig. 20 is a cross-sectional side view in partial section of the freeze-dry powder injector restoring device in Fig. 16, showing a cap assembly removed and a piston separated from a tube portion;
Fig. 21 is an enlarged perspective view in partial section of the freeze-dry powder injector restoring device in Fig. 16; and
Fig. 22 is an operational and cross-sectional side view in partial section of the freeze-dry powder injector restoring device in Fig. 16, shown in use.

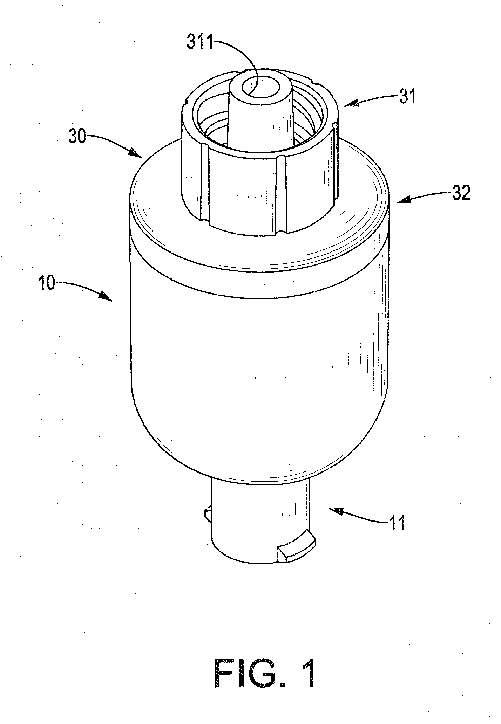

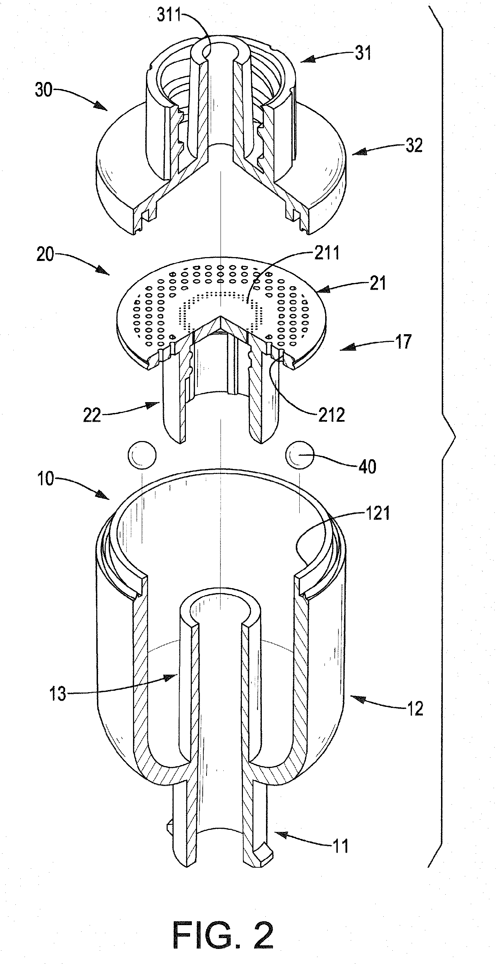

With reference to Figs. 1 and 2, a first embodiment of a freeze-dry powder injector restoring device in accordance with the present invention includes a container 10, a piston 20, a top cover 30, and a mixer 40.

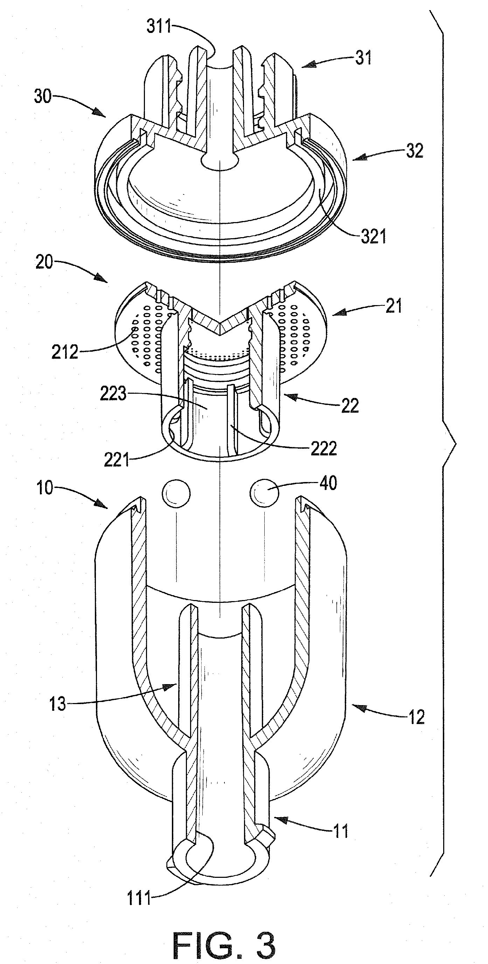

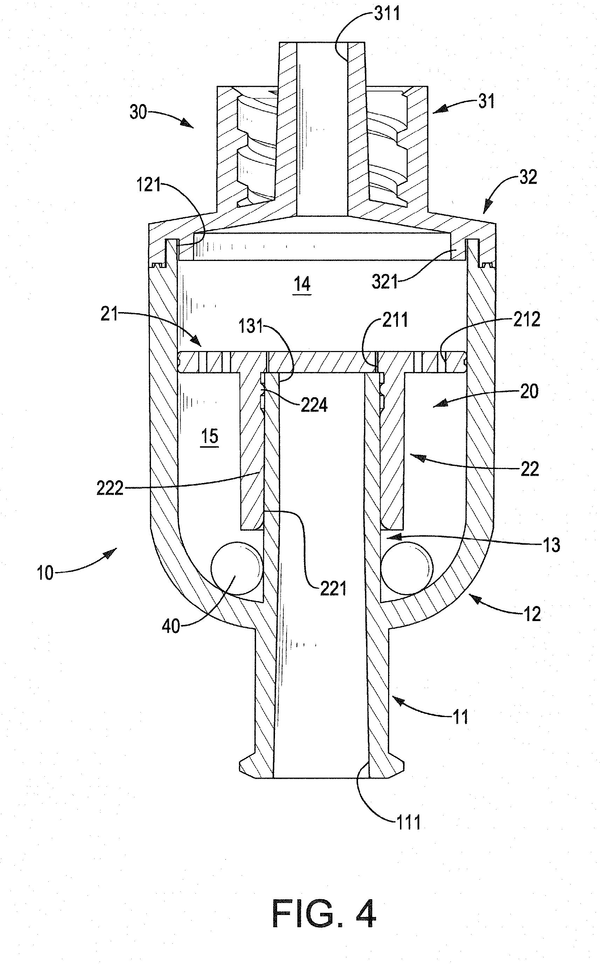

With reference to Figs. 2 to 5, the container 10 has a lower connecting portion 11, a cup body 12, and a tube portion 13. The lower connecting portion 11 is located at an end of the container 10, and the cup body 12 is located at another end of the container 10. The lower connecting portion 11 may be a Luer slip and has a lower opening 111. The cup body 12 forms an inner space and an upper opening 121 which fluidly communicates with the inner space. The tube portion 13 is mounted in the cup body 12. An end of the tube portion 13 fluidly communicates with the lower opening 111, and another end of the tube portion 13 forms a tube opening 131. In the first embodiment, the container 10 may be transparent and marked with scales of volume, but it is not limited thereto, as a configuration of the container 10 may be altered according to need.

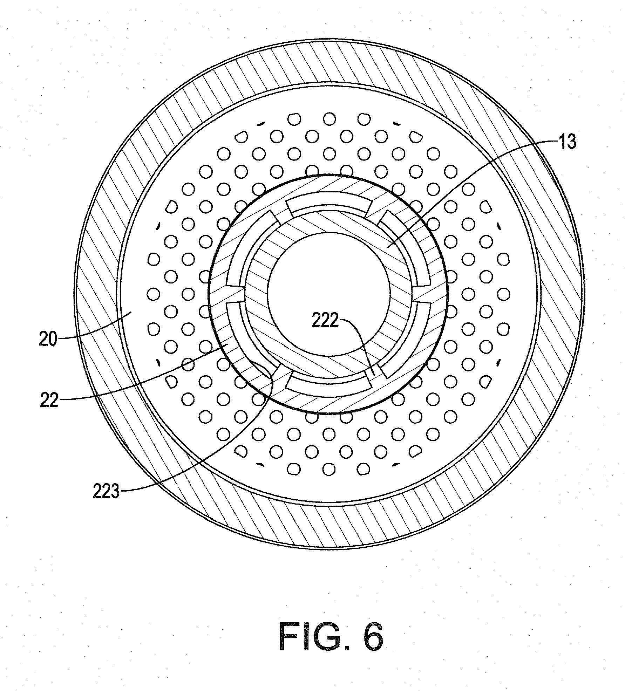

With reference to Figs. 2 to 6, the piston 20 may be transparent. The piston 20 has a separating board 21 and a connecting sleeve 22. The separating board 21 is a disk in the first embodiment. The piston 20 is mounted in the cup body 12 of the container 10 and is disposed on the tube portion 13, and the piston 20 divides the inner space of the cup body 12 into a first chamber 14 and a second chamber 15. The first chamber 14 is formed between the upper opening 121 and the piston 20. The second chamber 15 is formed between a bottom part of the cup body 12 and the piston 20. Multiple communicating channels 17 are formed on the piston 20 and disposed between the first chamber 14 and the second chamber 15, and the first chamber 14 and the second chamber 15 selectively and fluidly communicate with each other via the communicating channels 17. In the first embodiment, the communicating channels 17 include multiple gas releasing channels 211 and multiple filtering channels 212 formed through the separating board 21 at spaced intervals. The filtering channels 212 are disposed outward with respect to the gas releasing channels 211 on the separating board 21. Gas is allowed to pass the gas releasing channels 211 while liquid is not, and likely, liquid is allowed to pass the filtering channels 212 while the freeze-dried medicinal powder is not, but it is not limited to be holes or gaps, and any other structure or material that has the same filtering effect may be utilized.

The connecting sleeve 22 is tubular in shape, and the connecting sleeve 22 protrudes from a side of the separating board 21. The connecting sleeve 22 has a communicating opening 221, and the communicating opening 221 fluidly communicates with each one of the gas releasing channels 211. Multiple ribs 222 protrude from an inner wall surface of the connecting sleeve 22. The ribs 222 extend along an axial direction of the connecting sleeve 22, and the ribs 222 are spaced apart from each other. Every adjacent two of the ribs 222 form a groove 223 between them. A leakage resisting ring 224 protrudes from the inner wall surface of the connecting sleeve 22, the leakage resisting ring 224 is located at an end of the connecting sleeve 22, and said end of the connecting sleeve 22 is disposed adjacent to the separating board 21. To be more precise, the leakage resisting ring 224 surrounds and protrudes from the inner wall surface of the connecting sleeve 22.

The piston 20 is slidably mounted on the tube portion 13 and located adjacent to the tube opening 131. To be more precise, the connecting sleeve 22 of the piston 20 is sleeved on the tube portion 13, and thereby the piston 20 is capable of moving along the tube portion 13 via the connecting sleeve 22. The first chamber 14 is located between the upper opening 121 and the separating board 21, and the second chamber 15 is located between the tube portion 13 and the separating board 21. The ribs 222 together surround the tube portion 13. The leakage resisting ring 224 fits the tube portion 13, but it is not limited thereto. In another embodiment, the connecting sleeve 22 may not have the leakage resisting ring 224, but directly abuts an edge of the tube opening 131 of the tube portion 13 via the separating board 21.

The top cover 30 is connected to the container 10 and includes an upper connecting portion 31 and a covering portion 32. The upper connecting portion 31 is located at a side of the top cover 30, and the covering portion 32 is located at another side of the top cover 30 and is connected to the upper connecting portion 31. The upper connecting portion 31 has a connecting opening 311, and the upper connecting portion 31 may be a Luer lock. The covering portion 32 surrounds and protrudes from an outer wall surface of the upper connecting portion 31. The connecting opening 311 is formed through the covering portion 32. A blocking protrusion 321 protrudes from a surface of the covering portion 32, said surface of the covering portion 32 is away from the upper connecting portion 31, and the blocking protrusion 321 is arranged along a circumferential direction of the covering portion 32. The top cover 30 is fixed at and covers the upper opening 121 of the cup body 12 via the covering portion 32, and the blocking protrusion 321 is located in the first chamber 14.

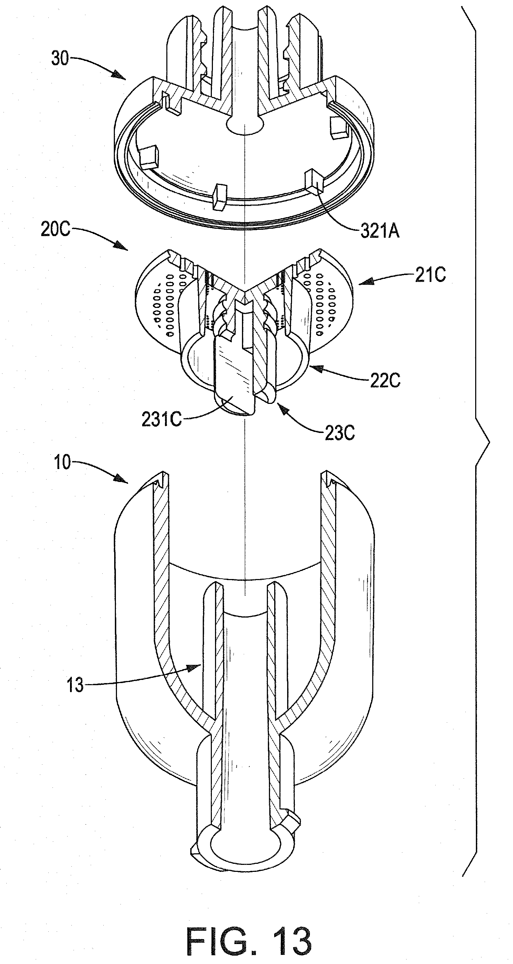

When the piston 20 moves upwardly relative to the container 10 and the top cover 30, the blocking protrusion 321 may be abutted by the separating board 21, and the connecting opening 311 fluidly communicates with the first chamber 14. In other words, the first chamber 14 is a space surrounded by the top cover 30 and the separating board 21 of the piston 20. In the first embodiment, the top cover 30 may be transparent, but it is not limited thereto, as a configuration of the top cover 30 may be altered according to need. In another embodiment, the covering portion 32 may not have the blocking protrusion 321, but directly abuts the separating board 21, or the blocking protrusion 321A may be formed by multiple ribs spaced apart from each other as shown in Fig. 13.

The mixer 40 is at least one ball and is disposed in the cup body 12. The mixer 40 may be a steel bead, but it is not limited thereto, as a configuration of the mixer 40 may be altered according to need. In another embodiment, the mixer 40 may be omitted.

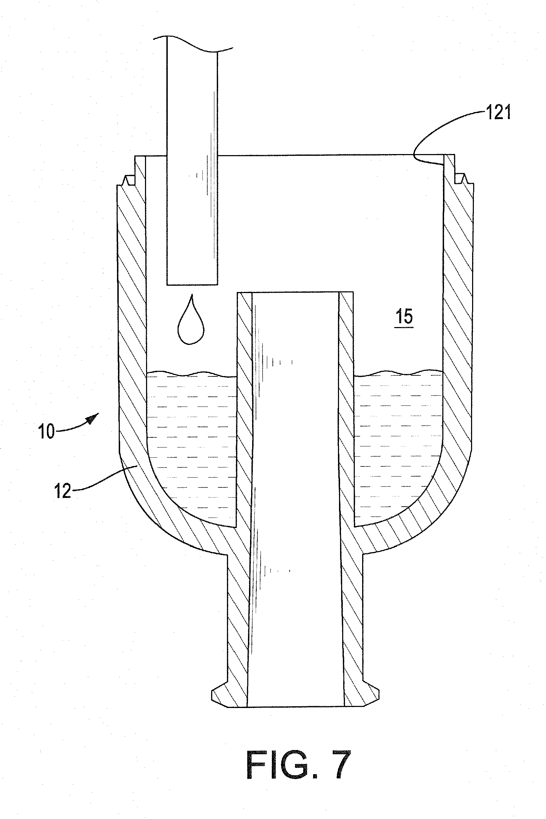

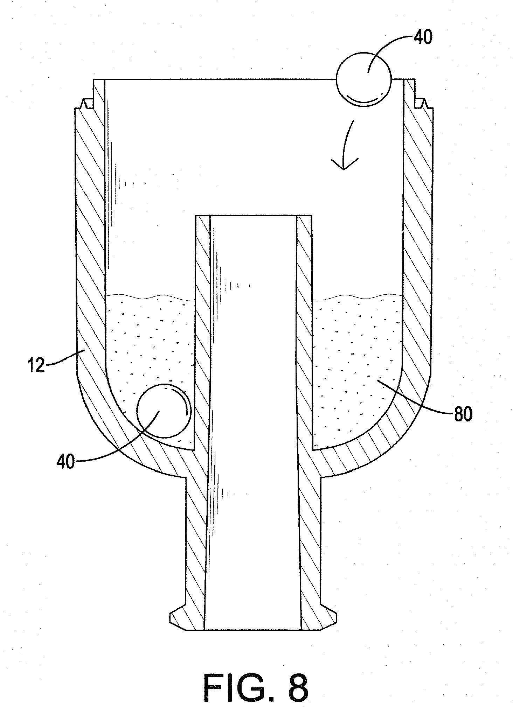

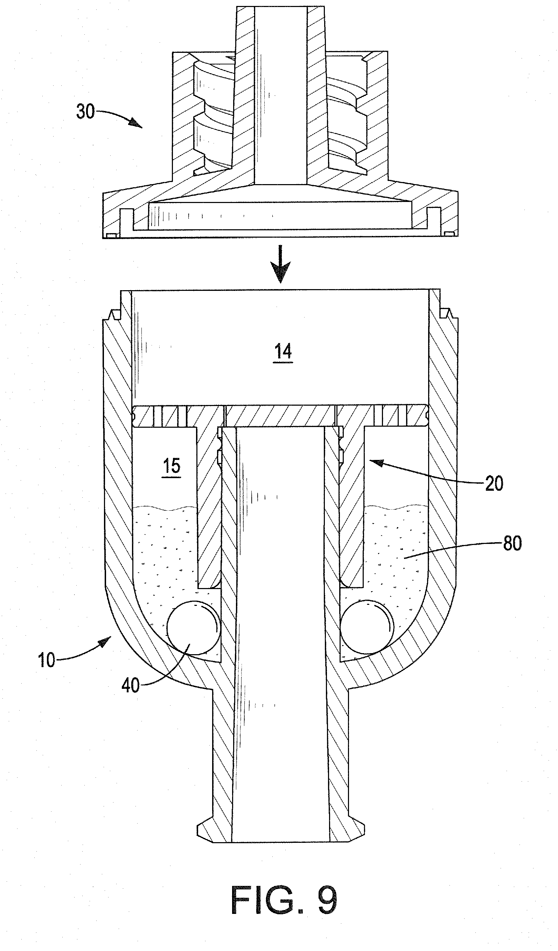

With reference to Fig.7, the container 10 is filled with a liquid state injection via the upper opening 121, and then the container 10 as well as the liquid state injection are sent to a freeze-drying process. With reference to Fig. 8, the liquid state injection will be freeze-dried and turns into a freeze-dried medicinal powder 80 retained in the cup body 12, and the mixer 40 is put into the cup body 12. With reference to Fig. 9, the piston 20 is installed in the container 10, and the top cover 30 covers the container 10 such that the freeze-dried medicinal powder 80 is sealed in the container 10. In the first embodiment, the top cover 30 is preferably fixed on the container 10 by ultrasonic welding, and then the freeze-dry powder injector restoring device may be vacuum packaged with aluminum foil for delivery.

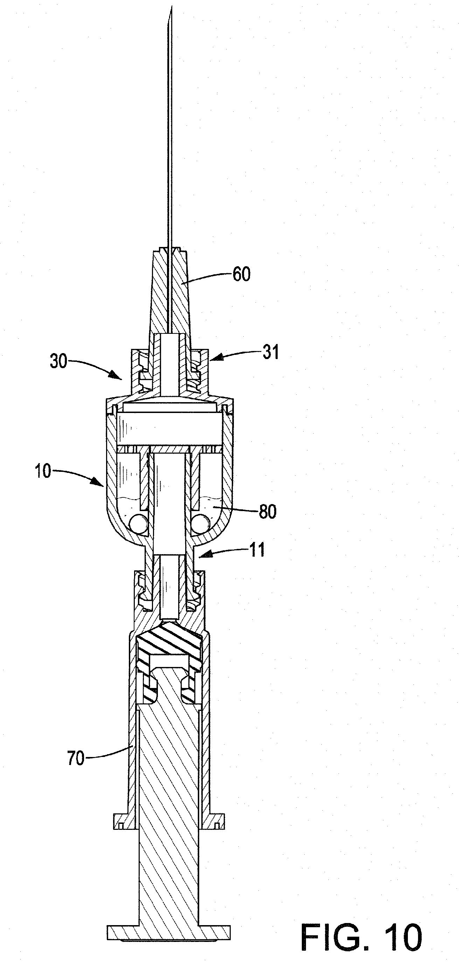

With reference to Fig. 10, a needle 60 and a syringe 70 may be applied together to use with the freeze-dry powder injector restoring device. The needle 60 is sleeved on the upper connecting portion 31 of the top cover 30, and said needle 60 may be a safety needle. The syringe 70 is inserted and fixed at the lower connecting portion 11 of the container 10 after sucking enough amount of solvent such as saline (aqueous sodium chloride).

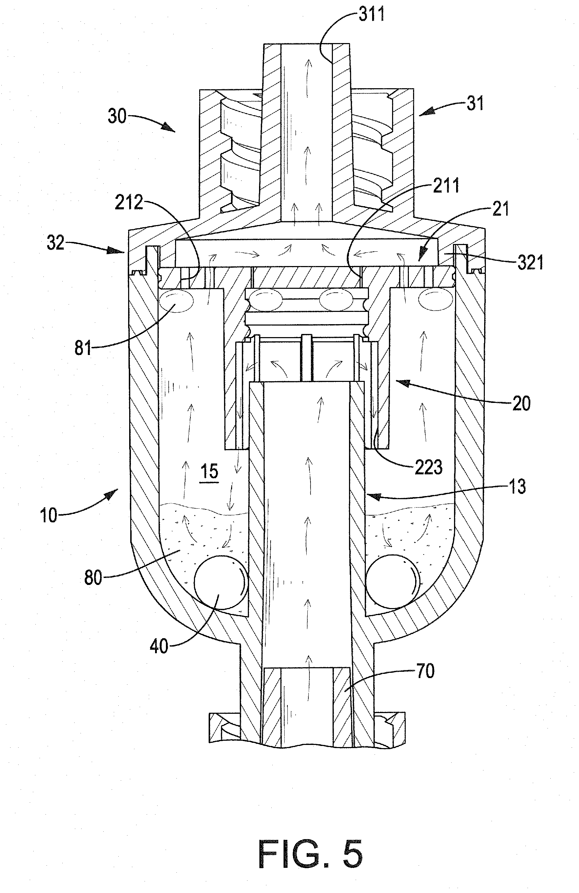

With reference to Figs. 5 and 10, to dissolve the freeze-dried medicinal powder 80, set the tip of the needle 60 upward and input the solvent from the syringe 70 to the tube portion 13. The syringe 70 keeps pumping in the solvent to push and move the piston 20 upward until the separating board 21 abuts the blocking protrusion 321 of the top cover 30. Then each one of the grooves 223 fluidly communicates with the tube portion 13. The solvent then flows to a bottom of the second chamber 15 via the grooves 223 to fill the second chamber 15, when a liquid level of the solvent in the second chamber 15 reaches a certain height, the freeze-dried medicinal powder 80 is dissolved in the solvent. A user may shake the freeze-dry powder injector restoring device to roll the mixer 40 in the second chamber 15, to help the freeze-dried medicinal powder 80 dissolve more quickly and completely. Besides, bubbles 81 may be generated in the tube portion 13 and the first chamber 14 when the solvent is fed in, and the bubbles 81 may pass the gas releasing channels 211 and the filtering channels 212 and be released via the connecting opening 311 of the upper connecting portion 31 when the syringe 70 pumps the solvent into the container 10.

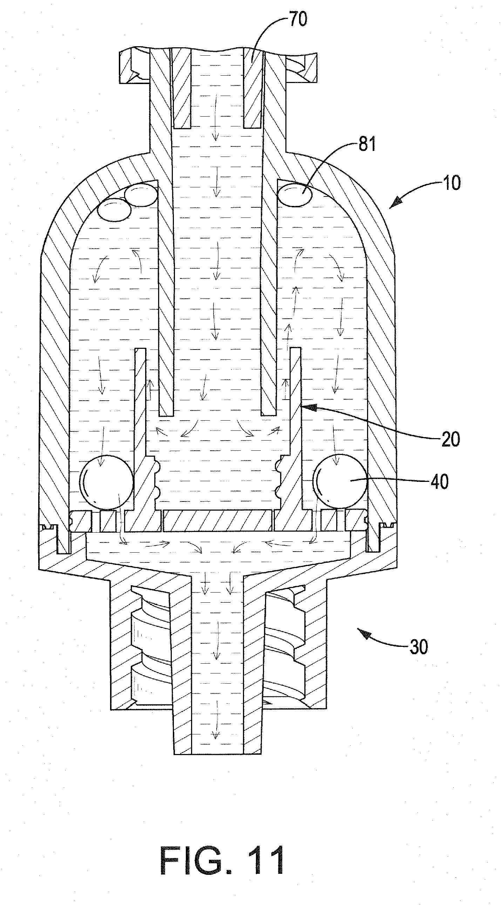

With reference to Figs. 5 and 11, after the freeze-dried medicinal powder 80 fully dissolves and the bubbles 81 are eliminated completely, flip the freeze-dry powder injector restoring device to turn the tip of the needle 60 downward to get ready to inject. If air residues exist in the container 10, the air residues would stay at the bottom of the container 10, and thus the air residues would not be injected into a human body.

Since the freeze-dried medicinal powder 80 has been accommodated in the container 10, the freeze-dry powder injector restoring device is capable of directly combining with the needle 60 and the syringe 70, such that procedures of dissolving the freeze-dried medicinal powder 80 can be simplified to increase convenience and efficiency of injection for patients or medical personnel, and it is convenient for the patients or medical personnel to carry the freeze-dried medicinal powder 80 in the freeze-dry powder injector restoring device. The process, steps, time, and manpower needed for dissolving the freeze-dried medicinal powder 80 may be simplified or reduced thanks to the freeze-dry powder injector restoring device. In addition, because a quantity of the freeze-dried medicinal powder 80 is fixed, a risk of dosage misjudgment would be reduced. Moreover, when dissolving the freeze-dried medicinal powder 80, the user may shake the freeze-dry powder injector restoring device to roll the mixer 40 to accelerate dissolving.

As to the medicine manufacturer, the freeze-dry powder injector restoring device is easy to manufacture since every component of the freeze-dry powder injector restoring device is easy to make, and thus the freeze-dry powder injector restoring device has a better manufacturing yield. The freeze-dry powder injector restoring device is easy to sterilize to achieve a requirement of cleanness and sterility. In addition, the freeze-dry powder injector restoring device matches freeze-dried medicine producing procedures to meet a requirement of freeze-drying locally in the original container and assembling on the production line, and thereby a cost of medicine manufacturing can be reduced.

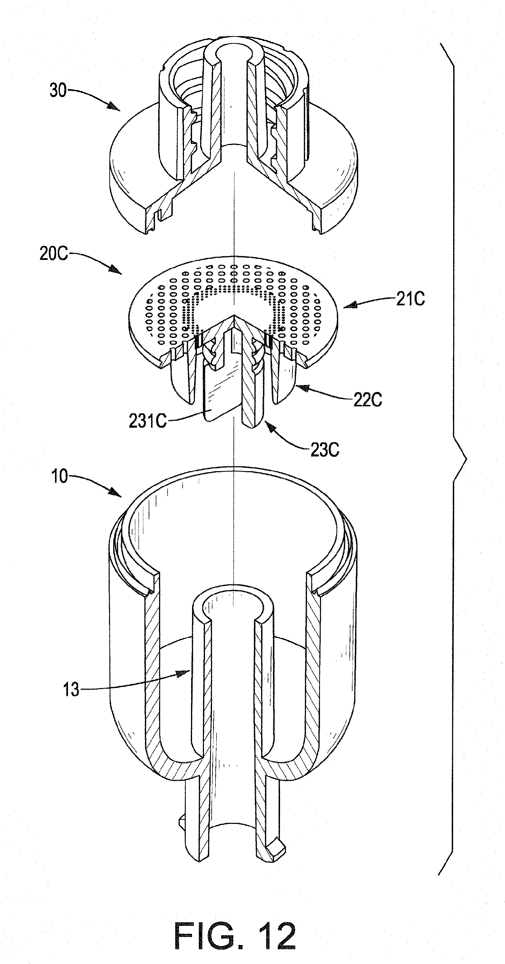

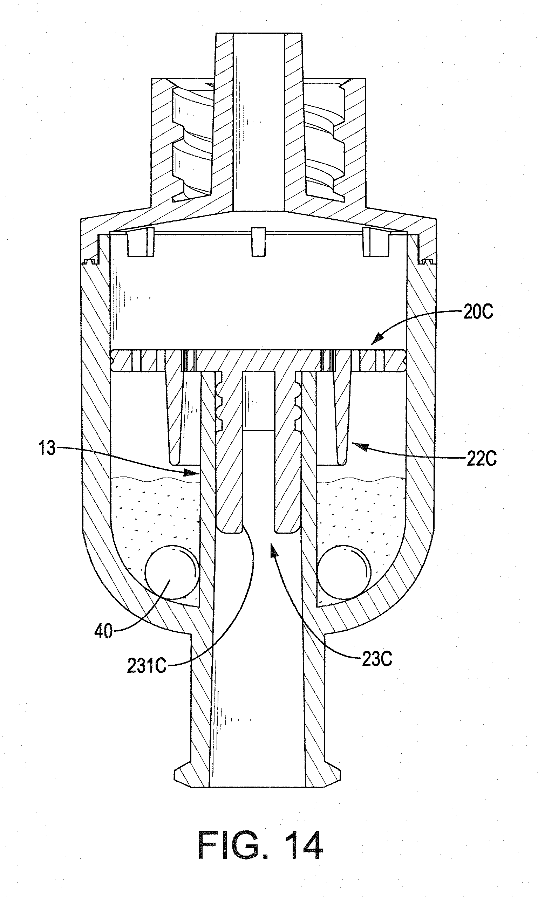

With reference to Figs. 12 to 14, a second embodiment of a freeze-dry powder injector restoring device in accordance with the present invention has the container 10, the top cover 30, and the mixer 40 being the same as the first embodiment, but the piston 20C of the second embodiment further has a split stopper 23C. To be more precise, an inner diameter of the connecting sleeve 22C is larger than the outer diameter of the tube portion 13, and the split stopper 23C is mounted on the separating board 21C and disposed in the connecting sleeve 22C. The split stopper 23C protrudes from the separating board 21C.

The split stopper 23C has two long slabs which are spaced apart from each other, and a stopper opening 231C is formed between the two long slabs. Shape of a cross-section of the split stopper 23C fits shape of a cross-section of an interior of the tube portion 13, such that the piston 20C may be mounted through the tube opening 131 of the tube portion 13 via the split stopper 23C, just like a plug stuck in the tube opening 131. As a result, the difference between the first embodiment and the second embodiment is that, the piston 20 in the first embodiment is installed by the connecting sleeve 22 sleeved on the tube portion 13, while the piston 20 in the second embodiment is installed by the split stopper 23C mounted in the tube portion 13.

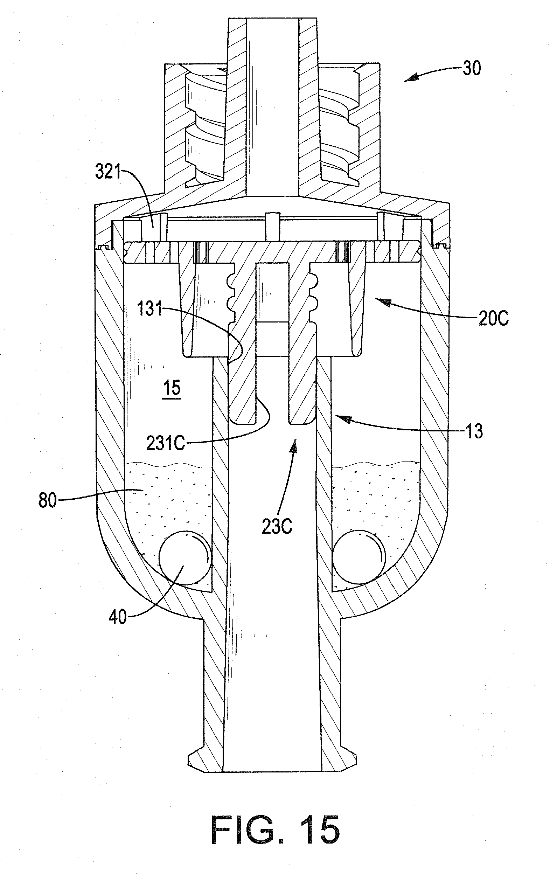

With reference to Fig. 15, the solvent pushes the piston 20C to move upward until the separating board 21C abuts the blocking protrusion 321 of the top cover 30, meanwhile the stopper opening 231C of the split stopper 23C is open to fluidly communicate with the second chamber 15, and thus the solvent is capable of flowing into the second chamber 15 via the stopper opening 231C. The solvent fills the second chamber 15 and is mixed with the freeze-dried medicinal powder 80, and then dissolves the freeze-dried medicinal powder 80. The follow-up process is the same as in the first embodiment, and thus is omitted.

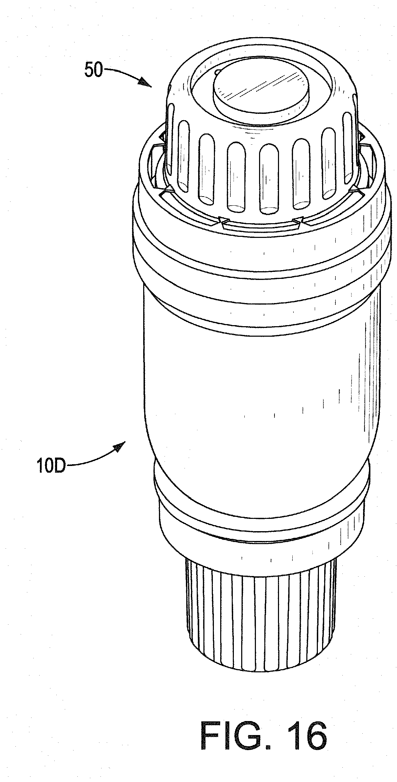

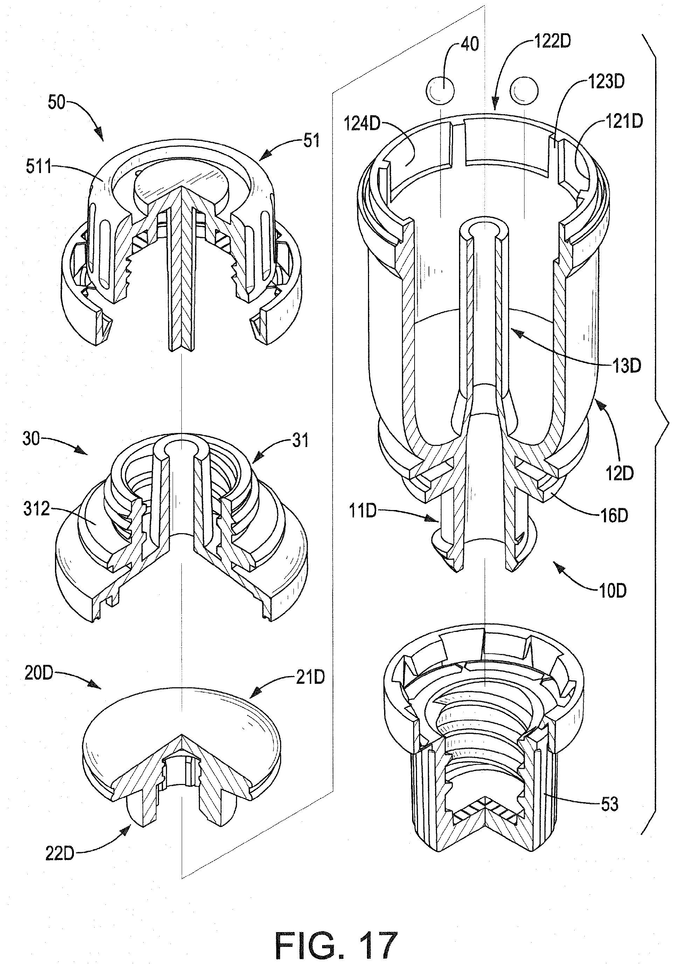

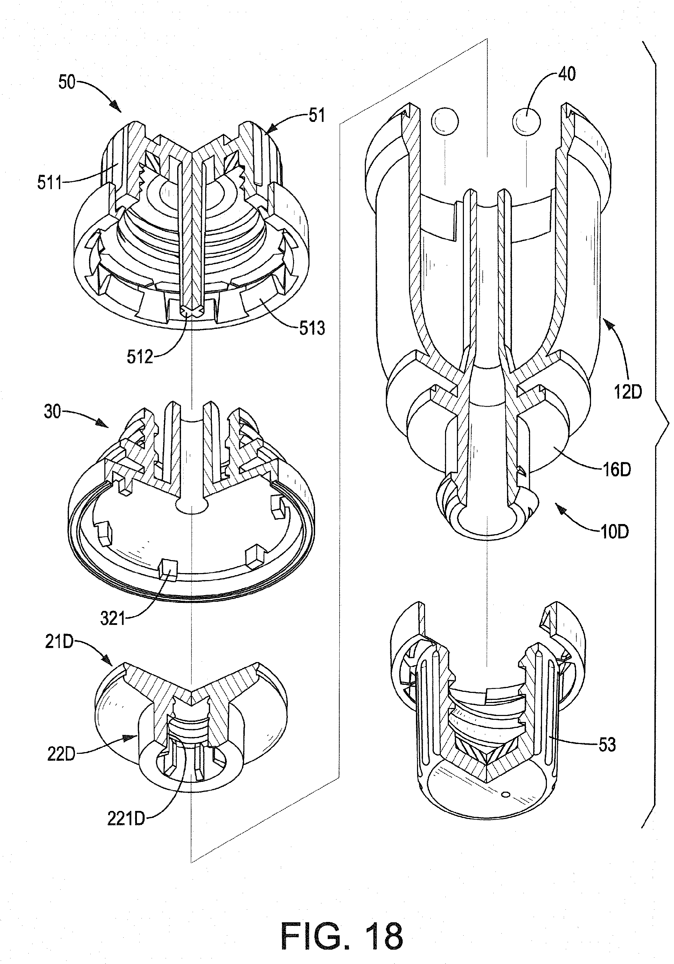

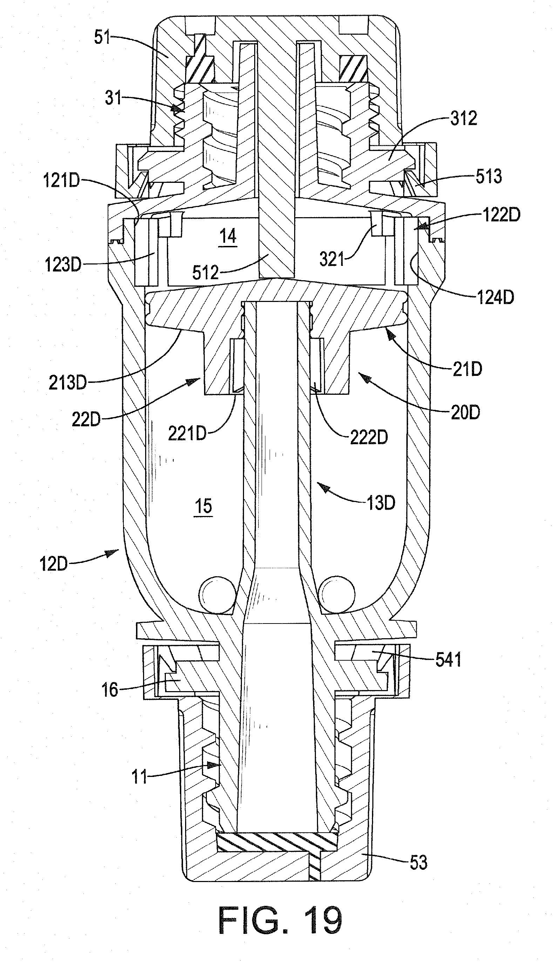

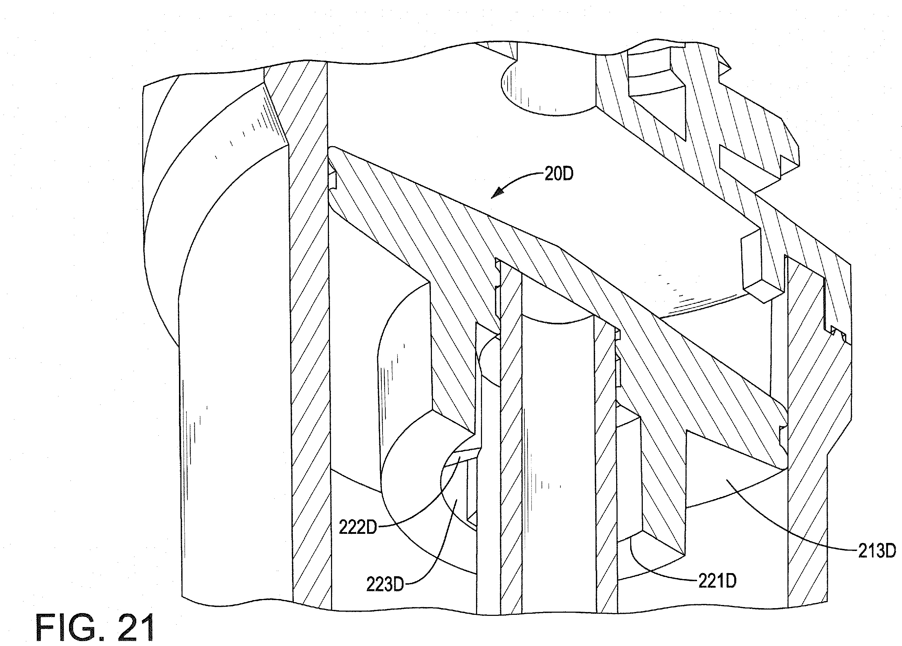

With reference to Figs. 16 to 22, a third embodiment of a freeze-dry powder injector restoring device has the top cover 30 and the mixer 40 being the same as the first embodiment, while the cup body 12D of the container 10D in the third embodiment further forms an annular connecting portion 122D and multiple trenches 124D.

The annular connecting portion 122D is recessed from an inner wall surface of the cup body 12D and located adjacent to the upper opening 121D. Each one of the trenches 124D is formed on the annular connecting portion 122D, to be more precise, multiple partitions 123D protrude from the annular connecting portion 122D, the partitions 123D are circumferentially arranged and spaced apart from each other, and thus each one of the trenches 124D is formed between adjacent two of the partitions 123D, but it is not limited thereto, as a configuration or a number of the trenches 124D may be altered according to need. Besides, the communicating channels 17 in the third embodiment are the trenches 124D.

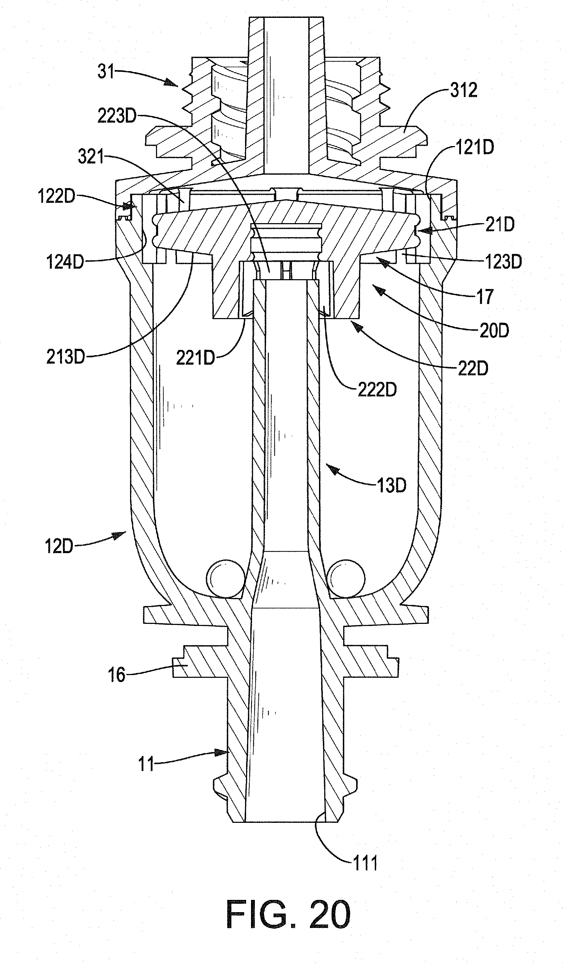

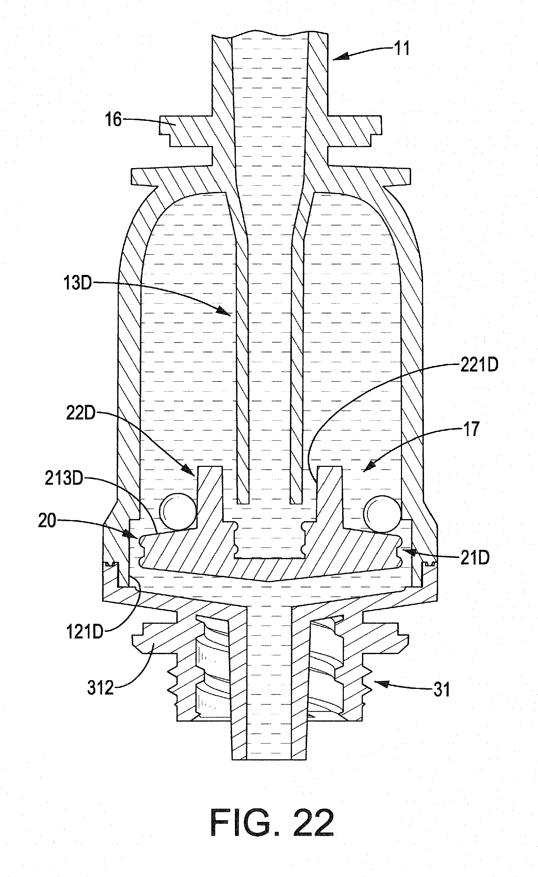

In the third embodiment, the separating board 21D of the piston 20D lacks the gas releasing channels 211 and the filtering channels 212, and a thickness of the separating board 21D is less than an axial length of the annular connecting portion 122D. The piston 20D has an oblique surface 213D adjacent to the connecting sleeve 22D, the oblique surface 213D may help reduce injection residues during injection, but it is not limited thereto. The connecting sleeve 22D also forms the multiple ribs 222D and the multiple grooves 223D. When the freeze-dry powder injector restoring device is not in use, the piston 20D is sleeved on the tube portion 13D via the connecting sleeve 22D, and the separating board 21D is beneath the annular connecting portion 122D. With reference to Figs. 20 and 22, when the freeze-dry powder injector restoring device is in use, the solvent pushes the piston 20D until the separating board 21D abuts the blocking protrusion 321 of the top cover 30, and thus each one of the grooves 223D is passable. Since each one of the trenches 124D fluidly communicates with the first chamber 14 and the second chamber 15, the solvent is capable of flowing through the grooves 223D, and flowing from the first chamber 14 to the second chamber 15 via the trenches 124D, and then the solvent may flow out of the freeze-dry powder injector restoring device via the upper connecting portion 31.

The freeze-dry powder injector restoring device may further have a cap assembly 50.

The cap assembly 50 includes an outlet cap 51 and an inlet cap 53. The outlet cap 51 has a cap part 511 and an abutting bar 512, and multiple outlet cap fixing units 513 protruding from an inner annular surface the cap part 511. The outlet cap fixing units 513 are arranged along a circumferential direction of the cap part 511, and the outlet cap fixing units 513 are spaced apart from each other. The abutting bar 512 protrudes from an inner bottom surface of the cap part 511. A screw thread is formed on an inner annular surface of the inlet cap 53, and multiple inlet cap fixing units 541 protrude from the inner annular surface of the inlet cap 53. To be more precise, the inner annular surface of the inlet cap 53 may be divided into a lower section and an upper section, the inlet cap fixing units 541 are located at the upper section of the inner annular surface of the inlet cap 53, and the screw thread is formed on the lower section of the inner annular surface of the inlet cap 53, but it is not limited thereto. The inlet cap fixing units 541 are arranged along a circumferential direction of the inlet cap 53, and the inlet cap fixing units 541 are spaced apart from each other.

In the third embodiment, to install the cap assembly 50, the upper connecting portion 31 further has an upper annular protrusion 312, and the upper annular protrusion 312 surrounds and protrudes from an outer wall surface of the upper connecting portion 31. Likely, the lower connecting portion 11D further has a lower annular protrusion 16D, and the lower annular protrusion 16D surrounds and protrudes from the outer wall surface of the lower connecting portion 11.

The outlet cap 51 covers on the upper connecting portion 31, and the abutting bar 512 is disposed through the connecting opening 311 and abuts the piston 20. Each one of the outlet cap fixing units 513 engages with the upper annular protrusion 312. Likely, the inlet cap 53 covers on the lower connecting portion 11, and each one of the inlet cap fixing units 541 engages with the lower annular protrusion 16D.

To open the freeze-dry powder injector restoring device, hold the cup body 12D and twist off the outlet cap 51 and the inlet cap 53, and then separate the abutting bar 512 from the connecting opening 311, and then the connecting opening 311 and the lower opening 111 are exposed for the needle 60 and the syringe 70 to connect.

The cap assembly 50 can help the user check whether the freeze-dry powder injector restoring device has been opened or not, but the configuration of the cap assembly 50 is not limited thereto. The freeze-dry powder injector restoring device may not have the upper annular protrusion 312, the lower annular protrusion 16D, and the cap assembly 50.

## Claims

1. A freeze-dry powder injector restoring device, **characterized in that** the freeze-dry powder injector restoring device comprises:
a container (10, 10D) having
a lower connecting portion (11, 11D) located at an end of the container (10, 10D), and the lower connecting portion (11, 11D) forming a lower opening (111);
a cup body (12, 12D) located at another end of the container (10, 10D), and the cup body (12, 12D) forming an inner space and an upper opening (121, 121D) fluidly communicating with each other; and
a tube portion (13, 13D) mounted in the cup body (12, 12D), an end of the tube portion (13, 13D) fluidly communicating with the lower opening (111), and another end of the tube portion (13, 13D) forming a tube opening (131);
a piston (20, 20C, 20D) mounted in the container (10, 10D), dividing the inner space into a first chamber (14) and a second chamber (15), and the piston (20, 20C, 20D) having
a separating board (21, 21C, 21D) and a connecting sleeve (22, 22C, 22D), the connecting sleeve (22, 22C, 22D) protruding from a side of the separating board (21, 21C, 21D), the connecting sleeve (22, 22C, 22D) mounted on the tube portion (13, 13D) and adjacent to the tube opening (131), and the connecting sleeve (22, 22C, 22D) capable of sliding along the tube portion (13, 13D); the connecting sleeve (22, 22C, 22D) having a communicating opening (221, 221D); and
multiple communicating channels (17) formed on the piston (20, 20C, 20D) and disposed between the first chamber (14) and the second chamber (15); and
a top cover (30) connected to the container (10, 10D) and having
an upper connecting portion (31) located at a side of the top cover (30); the upper connecting portion (31) forming a connecting opening (311) fluidly communicating with the first chamber (14); and
a covering portion (32) located at another side of the top cover (30), the covering portion (32) surrounding and protruding from an outer wall surface of the upper connecting portion (31); the top cover (30) fixed at the upper opening (121, 121D) of the cup body (12, 12D) via the covering portion (32); and
wherein, when the tube opening (131) is closed by the piston (20, 20C, 20D), the communicating opening (221, 221D) and each one of the communicating channels (17) is closed, and when the tube opening (131) is open, the tube opening (131), the communicating opening (221, 221D), the second chamber (15), each one of the communicating channels (17), and the first chamber (14) fluidly communicate with each other.

2. The freeze-dry powder injector restoring device as claimed in claim 1, wherein each one of the communicating channels (17) is disposed on the separating board (21, 21C, 21D) of the piston (20, 20C, 20D); the communicating channels (17) include
multiple gas releasing channels (211) formed through the separating board (21, 21C, 21D), and each one of the gas releasing channels (211) fluidly communicating with the communicating opening (221, 221D); and
multiple filtering channels (212) formed through the separating board (21, 21C, 21D), and the filtering channels (212) disposed outward on the separating board (21, 21C, 21D) with respect to the gas releasing channels (211).

3. The freeze-dry powder injector restoring device as claimed in claim 1, wherein
the cup body (12, 12D) forms an annular connecting portion (122D), the annular connecting portion (122D) is recessed from an inner wall surface of the cup body (12, 12D), and the annular connecting portion (122D) is located adjacent to the upper opening (121, 121D); and
the communicating channels (17) include multiple trenches (124D), and the trenches (124D) are formed on the annular connecting portion (122D).

4. The freeze-dry powder injector restoring device as claimed in claim 3, wherein the piston (20, 20C, 20D) has an oblique surface (213D), and the oblique surface (213D) is adjacent to the connecting sleeve (22, 22C, 22D).

5. The freeze-dry powder injector restoring device as claimed in any one of claims 1 to 4, wherein
the connecting sleeve (22, 22D) is tubular in shape;
the piston (20, 20D) is sleeved on the tube portion (13, 13D) via the connecting sleeve (22, 22D); and
the connecting sleeve (22, 22D) has
multiple ribs (222, 222D) protruding from an inner wall surface of the connecting sleeve (22, 22D), the ribs (222, 222D) extending along an axial direction of the connecting sleeve (22, 22D), and the ribs (222, 222D) spaced apart from each other; and
multiple grooves (223), and each one of the grooves (223) formed between every adjacent two of the ribs (222, 222D).

6. The freeze-dry powder injector restoring device as claimed in claim 5, wherein the connecting sleeve (22, 22D) has
a leakage resisting ring (224) protruding from the inner wall surface of the connecting sleeve (22, 22D), the leakage resisting ring (224) located at an end of the inner wall surface of the connecting sleeve (22, 22D), and said end adjacent to the separating board (21, 21D); the leakage resisting ring (224) fitting the tube portion (13, 13D).

7. The freeze-dry powder injector restoring device as claimed in claim 6, wherein the covering portion (32) has
a blocking protrusion (321) protruding from a surface of the covering portion (32), and said surface of the covering portion (32) away from the upper connecting portion (31); the blocking protrusion (321) arranged along a circumferential direction of the covering portion (32) and configured to abut the separating board (21, 21D).

8. The freeze-dry powder injector restoring device as claimed in claim 7, wherein the freeze-dry powder injector restoring device has at least one mixer (40) disposed in the cup body (12, 12D).

9. The freeze-dry powder injector restoring device as claimed in any one of claims 1 to 4, wherein
the connecting sleeve (22C) is tubular in shape, and an inner diameter of the connecting sleeve (22C) is larger than an outer diameter of the tube portion (13); and
the piston (20C) has
a split stopper (23C) mounted on the separating board (21C) and disposed in the connecting sleeve (22C), and the split stopper (23C) having a stopper opening (231C); the piston (20C) disposed through the tube opening (131) of the tube portion (13) via the split stopper (23C).

10. The freeze-dry powder injector restoring device as claimed in claim 9, wherein the covering portion (32) has
a blocking protrusion (321A) protruding from a surface of the covering portion (32), and said surface of the covering portion (32) away from the upper connecting portion (31); the blocking protrusion (321A) arranged along a circumferential direction of the covering portion (32) and configured to abut the separating board (21C).

11. The freeze-dry powder injector restoring device as claimed in claim 10, wherein the freeze-dry powder injector restoring device has at least one mixer (40) disposed in the cup body (12).
